# EUROPÄISCHE PATENTSCHRIFT

(11) **EP 0 436 886 B1**
(45) Veröffentlichungstag und Bekanntmachung des Hinweises auf die Patenterteilung: **25.05.1994**
(21) Anmeldenummer: 90124556.3
(22) Anmeldetag: 18.12.1990
(51) Int. Cl.: C12N 15/09, C12P 13/06, C07H 21/04, C12N 1/21

(54) **Verfahren zur Herstellung von L-Isoleucin und dafür geeignete Mikroorganismen und rekombinante DNA**
Process for producing L-isoleucine and microorganisms suitable therefore and recombinant DNA
Procédé de la production de L-isoleucine et micro-organismes propres à cela et ADN recombinant

(30) Priorität: 23.12.1989 DE 3942947
(43) Veröffentlichungstag der Anmeldung: 17.07.1991
(73) Patentinhaber: FORSCHUNGSZENTRUM JÜLICH GMBH, 52425 Jülich (DE)
(72) Erfinder: Möckel, Bettina, W-4000 Düsseldorf (DE); Cordes, Christiana, Dr., F-6700 Strassbourg (FR); Eggeling, Lothar, Dr., W-5170 Jülich (DE); Sahm, Hermann, Prof., W-5170 Jülich (DE)

(56) Entgegenhaltungen:
- EP-A- 0 131 171
- EP-A- 0 137 348
- EP-A- 0 204 326
- EP-A- 0 219 027
- EP-A- 0 233 581

## Beschreibung

Die Erfindung bezieht sich auf ein Verfahren zur Herstellung von L-Isoleucin durch Züchtung eines transformierten Mikroorganismus der Gattung Corynebakterium oder Brevibakterium in einem geeigneten Nährmedium und Isolierung von L-Isoleucin aus dem Medium, und sie umfaßt dafür geeignete Mikroorganismen und rekombinante DNA.

Die Aminosäure L-Isoleucin ist für Mensch und Tier essentiell und findet als Komponente verschiedener Nährgemische medizinischer Zweckbestimmung breite Verwendung. Ferner wird L-Isoleucin als Zugabe sowie als Reagenz für die pharmazeutische und chemische Industrie benutzt.

Es ist bekannt, daß sich mit Bakterien L-Isoleucin fermentativ aus Kohlehydraten wie Glukose, Fruktose, Hydrolysaten oder Melassen, sowie aus Vorstufen, wie 2-Hydroxybutyrat, 2-Ketobutyrat, Threonin oder 2-Aminobutyrat herstellen läßt. Dabei finden hauptsächlich Mutanten von Vertretern der Gattung Corynebacterium, Brevibacterium, oder Arthrobacter Verwendung, die gegen Aminosäureanaloga resistent sind. Diese Bakterien wurden durch klassische ungerichtete Mutagenese erhalten. Als Beispiel seien 2-amino-3-hydroxyvaleriansäure resistente Mutanten von Brevibakterien genannt (US-PS 4 329 427), sowie gegen 2-Amino-3-methylthiobuttersäure resistente Mutanten von Escherichia coli (JA-OS 69881/1978), oder gegen Trichloroalanin resistente Mutanten von Brevibakterien (JA-OS 35287/1979). Die Veränderungen der Mutanten können die Regulation von Enzymen betreffen, oder auch zu gesteigerter Enzymaktivität führen.

Durch die in den letzten Jahren entwickelten Methoden, DNA in vitro zu rekombinieren (r-DNA Technik) und coryneforme Bakterien zu transformieren, ist es möglich geworden Aminosäurebiosynthesegene in diesen Bakterien zu amplifizieren, und so durch erhöhte Enzymaktivität eine gesteigerte Aminosäurebildung zu erreichen. So wird in der EP-OS 0 137 348 ein Verfahren beschrieben, bei dem ein aus Brevibacterium isoliertes, für die Homoserinkinase kodierendes Gen (thrB) mit Vektor-DNA als rekombinante DNA in Brevibacterium wieder eingeführt wird und so die Produktion von L-Threonin und L-Isoleucin mit dem transformierten Organismus gesteigert wird. Gemäß dem US-PS 4 601 983 wird mit einem für Homoserindehydrogenase (hom) kodierenden Gen aus Brevibacterium mit Vektor DNA rekombinante DNA erzeugt, die in Brevibacterium eingeschleust wird, um so zu einer gesteigerten L-Threonin- und L-Isoleucinbildung zu gelangen. Gemäß der PCT-WO 88/09819 wird ein Verfahren beschrieben in dem neben den Genen die für die Homoserindehydrogenase und Homoserinkinase kodieren auch das Gen für die Threoninsynthase (thrC) isoliert und der Modifizierung unterworfen wird.

Die bislang genannten Gene (hom, thrB, thrC) sind sowohl für die L-Threonin-, als auch für die L-Isoleucinbiosynthese relevant, da L-Isoleucin in der Bakterienzelle aus L-Threonin gebildet wird. Es wurde nun gefunden, daß durch Überexpression von für die L-Isoleucinbiosynthese spezifischen Genen eine verbesserte L-Isoleucinproduktion erreicht werden kann. Das demgemäß entwickelte erfindungsgemäße Verfahren ist dadurch gekennzeichnet, daß man Mikroorganismen verwendet, die eine rekombinante DNA aus Vektor-DNA und für Threonindehydratase, Acetohydroxysäuresynthase und ggf. Isomeroreduktase kodierenden DNA-Sequenzen aufweist.

Gemäß der Erfindung werden mithin die für Threonindehydratase (ilvA), Acetohydroxysäuresynthase (ilvBN) und ggf. Isomeroreduktase (ilvC) kodierenden Gene aus Corynebakterien oder Brevibakterien isoliert, mit geeigneten Vektoren rekombiniert und schließlich Corynebakterien oder Brevibakterien mit der so erhaltenen rekombinanten DNA transformiert und für die Produktion von L-Isoleucin verwendet, um auf diese Weise zu einer gesteigerten L-Isoleucin Produktion zu gelangen. Die genannten Gene können einzeln oder in Kombination zur Erzeugung transformierter Mikroorganismen verwendet werden.

In Praxi kann die Isolation der genannten Gene durch jede der bekannten Methoden zur Genklonierung ausgeführt werden (Maniatis, T. et al., "Molecular Cloning", Gold Spring Harbor Laboratory, 1982), wobei die nachfolgend genannte Methode, die Komplementation definierter Escherichia coli Mutanten, die bevorzugte ist. Zunächst wird chromosomale DNA aus einem Corynebacterium oder Brevibacterium isoliert, das die für Threonindehydratase (ilvA), Acetohydroxysäuresynthase (ilvBN), oder Isomeroreduktase (ilvC) kodierende Sequenz enthält. Geeignete Bakterien sind zum Beispiel die Wildtypstämme Corynebacterium glutamicum ATCC 13032, oder Brevibacterium flavum ATCC 13826. Da die Menge und Aktivität der Acetohydroxysäuresynthase in Corynebakterien durch Isoleucin reduziert wird (Eggeling et al., 1987, Appl Microbiol Biotechnol 25: 346-351) sowie ebenfalls die Aktivität der Threonindehydratase durch L-Isoleucin, sind als Donatoren chromosomaler DNA auch Stämme geeignet, in denen die Repression der Genexpression sowie die Inhibition der Enzymaktivität nicht mehr erfolgt, wie dies zum Beispiel in dem hinterlegten Stamm DSM5659 der Fall ist. Zur Spaltung der chromosomalen DNA können verschiedene Restriktionsenzyme eingesetzt werden.

Als Vektor-DNA werden Plasmide, Phagen oder Derivate davon benutzt, die aus Bakterien der Gruppe der Corynebakterien oder Brevibakterien stammen, wie z.B. pZ1, pJC1, pSA77, pBL1. Mit diesen Vektoren ist die Komplementation von Corynebakterien oder Brevibakterien möglich, die in ilvC, ilvBN oder ilvA defekt sind. Um durch Komplementation von Escherichia coli Mutanten, die in ilvC, ilvBN, oder ilvA defekt sind, die Gene zu isolieren, wird die gespaltene chromosomale DNA mit E. coli Vektoren oder Cosmiden ligiert und in einem geeigneten E. coli Stamm zunächst eine Genbank erstellt. Dabei wird z.B. mit dem Restriktionsenzym Sau3A gespaltene DNA mit dem BamH1 gespaltenen Cosmid pHC79 ligiert, und die resultierenden rekombinanten Fusionsplasmide durch Transduktion (Hohn et al., 1980, Gene 11:291) in- z.B.- den E. coli Stamm DH5 eingeführt (Hanahan, 1985, "DNA Cloning", vol.1, IRL Press). Mit den so erhaltenen Fusionsplasmiden wird eine E. coli Mutante transformiert, die L-Isoleucin zum Wachstum benötigt, wie z.B. E. coli CGSC 5769 (Lawther et al., 1982, J Bacteriol 149: 294) die in den Acetohydroxysäuresynthase-Genen defekt ist und folglich ohne L-Isoleucin Supplementation nicht wachsen kann. Solche Transformanten, die nun ohne L-Isoleucin Supplementation wachsen können werden isoliert. Sie besitzen Acetohydroxysäuresynthase-Aktivität und enthalten rekombinante DNA, die im Falle der Komplementation von E. coli CGSC 5769 für das gewünschte Acetohydroxysäuresynthasegen (ilvBN) aus Corynebacterium glutamicum codiert. Um das Gen aus dem Fusionsplasmid zu isolieren werden die Bakterien z.B. mit SDS lysiert und mit Phenol behandelt. Dann wird ein zweifaches Volumen Ethanol zugegeben wodurch die r-DNA präzipitiert.

In gleicher Weise, wie vorstehend detailliert für das Gen der Acetohydroxysäuresynthase (ilvBN) beschrieben, kann auch das Gen für die Isomeroreduktase (ilvC) oder für die Threonindehydratase (ilvA) durch Komplementation der geeigneten E. coli Mutanten, wie z.B. Stamm CU406 (ilvA) (Gayda et al., 1971, J. Bacteriol. 142: 556) oder Stamm JP58 (ilvC) (Butlin et al., 1971, Biochem J 124:75) isoliert werden. Anschließend wird das isolierte, für die Isoleucinbiosynthese codierende Gen in einen entsprechenden Vektor integriert, was durch Verdau des Vektors mit den entsprechenden Restriktionsenzymen und Ligation des Vektors mit dem ebenfalls entsprechend restringierten Gen erfolgt.

Die Bildung von Plasmiden, die ilvA, ilvBN und ggf. ilvC enthalten, erfolgt stufenweise durch aufeinanderfolgenden Einbau der einzelnen Gene.

Der Einbau des Plasmids, das ilvA, ilvBN und ggf. ilvC enthält, in Coryne- oder Brevibakterien kann durch Transformation von Protoplasten oder Sphäroplasten nach einem modifizierten Verfahren erfolgen (Santamaria et al. 1985, J Bacteriol 162: 463), durch Elektroporation (Wolf et al. 1989, Appl Microbiol Biot 30: 283), oder durch Konjugation mittels mobilisierbarer Vektoren (Simon et al. 1986, Methods in Enzymology 118: 640). Als Rezipienten der r-DNA, werden bevorzugt mutierte Stämme von C. glutamicum verwendet, die bereits L-Isoleucin ins Kulturmedium ausscheiden. Repräsentative Beispiele solcher Rezipienten sind gegen L-Isoleucinanaloga (z.B. Isoleucinhydroxamat, 2-Amino-3-hydroxyvaleriansäure) resistente Stämme von C. glutamicum oder B. flavum. Transformanten können leicht aufgrund der im Vektor lokalisierten Resistenz, wie z.B. in pJC1 der Kanamycin-Resistenz, selektioniert werden. Die plasmidcodierten Gene führen zu erhöhter Aktivität der Isoleucinbiosyntheseenzyme. Dies wurde für die Acetohydroxysäuresynthase nach dem Verfahren von Umbarger et al. 1958, J Biol Chem 233: 1156, für die Isomeroreduktase nach dem Verfahren von Arfin et al. 1969, J Biol Chem 244: 1118, und für die Threonindehydratase nach dem Verfahren von Umbarger et al., 1958, J Biol Chem 233: 1156 nachgewiesen.

Die Methoden zur Kultivierung der nach dem beschriebenen Verfahren hergestellten Bakterien sind ähnlich den bekannten Methoden wie sie für L-Isoleucin produzierende Bakterien benutzt werden. Die benutzten Kulturmedien können konventionelle Medien sein, die Kohlenstoffquellen, Stickstoffquellen und organische Ionen, und gegebenenfalls organische Bestandteile wie Vitamine oder Aminosäuren enthalten. Beispiele geeigneter Kohlenstoffquellen sind Glukose, Saccharose, Laktose, Stärkehydrolysate oder Molassen. Als Stickstoffquelle können gasförmiges Ammonium, wässriges Ammonium, Ammoniumsalze und andere Stickstoff enthaltenden Materialien benutzt werden.

Die Kultivierung der rekombinanten Bakterien, die die überexprimierten L-Isoleucinbiosynthesegene enthalten erfolgt unter aeroben Bedingungen, wobei pH und Temperatur des Mediums in einem günstigen Bereich gehalten werden, und wird fortgeführt bis die Bildung von L-Isoleucin aufhört.

Die Isolierung und Reinigung der Aminosäure aus dem Medium erfolgt mittels bekannter Methoden.

Es zeigt sich, daß durch das in der vorliegenden Erfindung beschriebene Verfahren mehr L-Isoleucin gebildet werden kann.

### Beispiel 1

### Präparation chromosomaler DNA, die ein Isomeroreduktasegen (ilvC) enthält

Zellen des Stammes Corynebacterium glutamicum ATCC 13032 wurden 18 Stunden bei 30 °C in 100ml CgIII Medium kultiviert, welches folgende Zusammensetzung hat: 10 g/l Pepton aus Fleisch, 10 g/l Hefeextrakt und 5 g/l NaCl. Die durch Zentrifugation vom Kulturmedium abgetrennten Zellen wurden zweimal mit TE-Puffer gewaschen und in 10 ml desselben Puffers aufgenommen, der 10 mmol/l Tris-HCl, 1 mmol/l EDTA, pH7,9 ist. Nach Zugabe von 100 mg Lysozym zu den resuspendierten Zellen wurde 8 Stunden bei 30°C inkubiert, 2 mg Proteinase K zugefügt, und dann 18 Stunden bei 50°C inkubiert. Anschließend wurde 1 ml 10% Laurylsarkosinat hinzugegeben, und schließlich zum nun klaren Lysat 20 ml -20°C kaltes Äthanol zugefügt, wodurch die chromosomale DNA ausfiel, die mit Hilfe eines Glasstabes aus der Lösung herausgedreht wurde. Die DNA wurde mit 70%igem Äthanol gewaschen, und nach dem Trocknen in TE-Puffer aufgenommen. 0,1 µg dieser DNA wurden mit 0,1 U des Restriktionsenzyms Sau3A (Hersteller Boehringer, Mannheim) in 10 mmol/l Tris-HCl, 75 mmol/l NaCl, 10 mmol/l MgCl₂, pH 7,2 bei 37°C 1 Stunde inkubiert. Nach dieser Inkubationszeit wurde die Reaktion durch Zugabe von 20 mmol/l EGTA und 10-minütiges Erhitzen auf 65 °C gestoppt.

Der Vektor pBR322 wurde aus dem E. coli Stamm DH5 wie in Maniatis et al. (1982) Molecular Cloning, Cold Spring Harbour Laboratory beschrieben, isoliert. 0,1 µg des Vektors wurde mit 1U des Restriktionsenzyms BamH1 in 10 mmol/l Tris-HCl, 100mmol/l NaCl, 5 mmol/l MgCl₂, 1 mmol/l 2-Mercaptoethanol, pH 8 ,37 °C 40 Minuten inkubiert. Dann wurden 5 U alkalische Phosphatase zugegeben und weitere 20 Minuten inkubiert. Die Reaktion wurde durch EGTA und Erhitzen gestoppt, wie zuvor für den Verdau der chromosomalen DNA beschrieben. Beide Verdaus wurden mit 500 µl einer mit 0,1 mol/l Tris-HCl, pH 8 gesättigten Phenollösung extrahiert und anschließend die wässrige Phase von der Phenol-haltigen Phase durch Zentrifugation getrennt. Der wässrige Überstand wurde anschließend mit 500 µl Chloroform/Isoamylalkohol (24:1, v/v) extrahiert. Der resultierende wäßrige Überstand wurde mit 0,25 Volumenteilen 2 mol/l Lithiumchlorid und 2,5 Volumenteilen Äthanol versetzt und die dadurch präzipitierte DNA in 10µl 10 mmol/l Tris-HCl, 1 mmol/l EDTA pH 7.6 aufgenommen. Zur Ligation wurde 1 µl der chromosomalen DNA mit 1 µl des pBR322 Präzipitats zusammengegeben und 8 µl 2 mmol/l Tris-HCl, 10 mmol/l MgCl₂, 10 mmol/l Dithiothreitol, 0,6 mmol/l Adenosintriphosphat, pH7,6 sowie 1 U T4 Ligase hinzugefügt. Die Mischung wurde 16 Stunden bei 12 °C inkubiert.

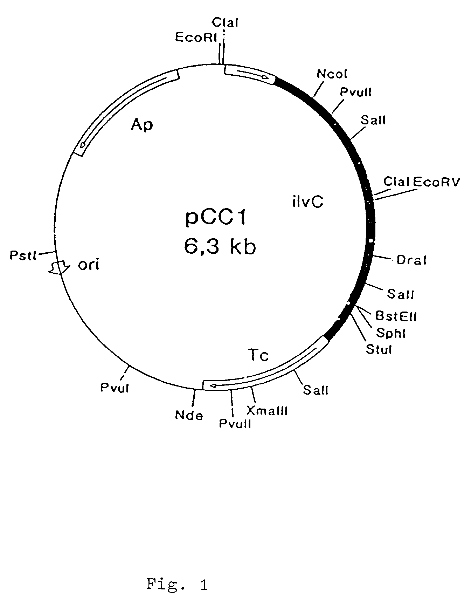

Mit diesem Ligase Reaktionsansatz wurde E. coli DH5 wie bei Hanahan beschrieben transformiert (Hanahan, D. 1985, Techniques for transformation. In: DNA cloning ed Glover, IRL Press), und auf Ampicillin-haltigem Medium ausplattiert. Von etwa 6000 erhaltenen Transformanten wurde Plasmid DNA isoliert, und damit der Isoleucin benötigende im Isomeroreduktasegen (ilvC) defekte E.coli Stamm JP58, erneut wie bei D. Hanahan beschrieben, transformiert. Der Transformationsansatz wurde auf LB Medium plus 50 µg/ml Ampicillin ausplattiert und bei 37°C bebrütet. Die hochgewachsenen Transformanten wurden durch replica plating auf ein Minimalmedium Übertragen, das nur Wachstum von Kolonien erlaubt, die Isomeroreduktaseaktivität infolge des aus C. glutamicum in pBR322 ligierten chromosomalen DNA Fragments enthalten. Dieses Medium enthält 3 g K₂HPO₄, 6 g KH₂PO₄ und 6,8 g Yeast Nitrogen Base w/o Amino Acids (Hersteller Difco). Einige nicht mehr Isoleucin bedürftige Transformanten wurden erhalten. Deren Plasmid DNA wurde isoliert, mit verschiedenen Restriktionsenzymen verdaut und anschließend einer Agarosegel-Elektrophorese unterzogen. Die Restriktionskarte des Plasmids ist in Abbildung 1 dargestellt. Aus diesem Plasmid wurde nach bekannten Verfahren das für die Isomeroreduktase kodierende DNA-Fragment durch EcoR1/Xma3 Verdau isoliert, in den Pendelvektor pJC1 ligiert und mit 1 µg davon Protoplasten von C. glutamicum ATCC 13032 transformiert, wie in der DE-Patentanmeldung P 37 37 729.9 beschrieben. Die Isomeroreduktaseaktivität in dem so erhaltenen rekombinanten Stämmen wurde entsprechend dem Verfahren von Arfin et al., 1969, J Biol Chem 244: 1118-1127 bestimmt. Sie betrug 0,7U/mg Protein, wogegen sie im Ausgangsstamm 0,2 U/mg Protein beträgt.

### Beispiel 2:

### Präparation chromosomaler DNA die für Acetohydroxysäuresynthaseaktivität codiert (ilvBN) und Isomeroreduktaseaktivität codiert (ilvC).

Chromosomale DNA aus C. glutamicum ATCC 13032 wurde wie in Beispiel 1 beschrieben isoliert. Diese DNA wurde partiell verdaut, indem 0,1 µg DNA mit 0,02 U des Restriktionsenzyms Pst1 in 10 mmol/l Tris-HCl, 100 mmol/ NaCl, 10 mmol/l MgCl₂, 100 µg/ml Rinderserumalbumin, pH7,5 bei 37°C für 20 Minuten inkubiert wurde.

Als Vektor wurde das Cosmid pHC79 benutzt, das aus dem E. coli Stamm DH5 wie in Maniatis et al. (1982) Molecular Cloning, Cold Spring Harbour Laboratory beschrieben, isoliert wurde. 0,1 µg dieses Vektors wurden mit 1 U des Restriktionsenzyms Pst1 in dem gleichen Puffer wie für den Verdau der chromosomalen DNA angegeben linearisiert, indem der Ansatz 40 Minuten bei 37°C inkubiert wurde. Dann wurden 5 U alkalische Phosphatase zugegeben und weitere 20 Minuten inkubiert. Beide Verdaus wurden wie in Beispiel 1 angegeben mit Phenol/Chloroform extrahiert und in 10 µl 10 mmol/l Tris-HCl, 1 mmol/l EDTA pH7,6 aufgenommen. Zur Ligation wurde 1 µl der chromosomalen DNA und 1 µl des Cosmids zusammengegeben und wie in Beispiel 1 beschrieben ligiert.

Die resultierende DNA wurde in vitro in infektionsfähige Phagenköpfe verpackt, wozu der "DNA packaging Kit" des Untenehmens Boehringer Mannheim benutzt wurde, der die benötigten Phagenlysate enthält. Mit dem resultierenden Phagenlysat wurde der E. coli Stamm ED8654 infiziert. Dazu wurde der Stamm über Nacht in TB Medium (10 g/l Trypton, 8 g/l NaCl, 0,2% (w/v) Maltose, 5 mg/l Vitamin B₁) in 100 ml angezogen. Die abzentrifugierten Zellen wurden in 40 ml 10 mmol/l MgSO₄ resuspendiert und 45 Minuten bei 42°C belüftet. Diese Zellen wurden mit 5 µl Lysat infiziert, und auf Tetracyclin enthaltendem LB Medium ausplattiert (10 g/l Trypton, 5 g/l Hefeextrakt, 10 g/l NaCl, 20 µg/ml Tetracyclin).

Aus der so hergestellten Genbank chromosomaler DNA von C. glutamicum in E. coli ED8654 wurde Plasmid DNA isoliert. Mit dieser wurde wie bei D. Hanahan beschrieben der Isoleucin benötigende in den Acetohydroxysäuresynthasegenen defekte E. coli Stamm CGSC5769 transformiert. Der Transformationsansatz wurde auf LB Medium plus 20 µg/ml Tetracyclin ausplattiert und bei 37°C bebrütet. Die hochgewachsenen Transformanten wurden durch replica plating auf das Minimalmedium wie in Beispiel 1 angegeben übertragen, das nur Wachstum von Kolonien erlaubt, die Acetohydroxysäuresynthaseaktivität infolge des aus C. glutamicum in pHC79 ligierten chromosomalen DNA Fragmentes enthalten. Einige nicht mehr Isoleucin bedürftige Transformanten wurden erhalten. Deren Plasmid DNA wurde isoliert, mit verschiedenen Restriktionsenzymen verdaut und anschließend einer Agarosegel-Elektrophorese unterzogen. Die Restriktionskarte des DNA-Fragments ist in Abbildung 2 dargestellt. Mit diesem DNA-Fragment in den Pendelvektor pJC1 ligiert wurde C. glutamicum ATCC13032 wie in Beispiel 1 beschrieben transformiert, und die Acetohydroxysäuresynthaseaktivität entsprechend dem Verfahren von Umbarger und Brown (1958, J Biol Chem 233: 1156-1160) bestimmt. Sie betrug etwa 0,35 U/mg Protein, wogegen sie im Ausgangsstamm 0,02 U/mg Protein beträgt. Der das Plasmid pCC2-42 enthaltende Corynebacterium glutamicum Stamm ist bei der Deutschen Stammsammlung für Mikroorganismen unter der Nummer DSM5664 hinterlegt.

### Beispiel 3

### Präparation chromosomaler DNA die für Threonindehydrataseaktivität codiert (ilvA).

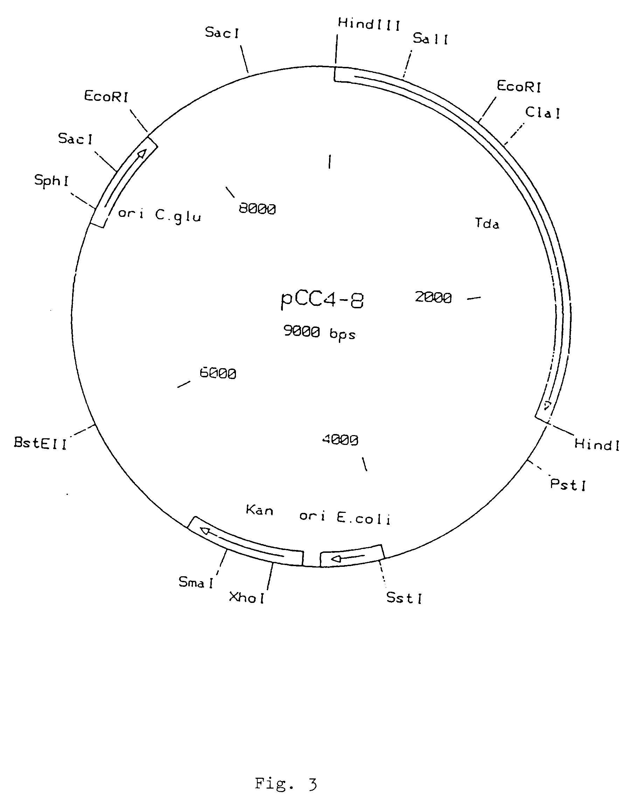

Die DNA wurde aus Zellen des Stammes C. glutamicum S9L2 wie in Beispiel 1 beschrieben isoliert. Dieser Stamm zeichnet sich dadurch aus, daß dessen Threonindehydrataseaktivität nicht mehr durch L-Isoleucin gehemmt wird, wie es im Ausgangsstamm ATCC13032 der Fall ist. Er ist bei der Deutschen Stammsammlung für Mikroorganismen unter der Nummer DSM5659 hinterlegt. Die chromosomale DNA (0,1µg) wurde mit 1 U des Restriktionsenzyms Hind3 für 1 Stunde bei 37°C in 10 mmol/l Tris-HCl, 50 mmol/l NaCl, 10 mmol/l MgCl₂, 14 mmol/l Dithiothreitol, pH7,6 verdaut. Sie wurde wie in Beispiel 1 beschrieben durch Phenol/Chloroform Extraktion aufgereinigt, und anschließend mit dem ebenfalls mit dem Restriktionsenzym Hind3 verdauten und aufgereinigten Vektor pUC18 (Vieira et al., 1982 Gene 19: 259) unter Bedingungen wie in Beispiel 1 beschrieben ligiert. Mit dem Ligationsansatz wurde der E. coli Stamm CU406 transformiert, und auf LB Medium plus 50 µ/l Ampicillin ausplattiert. Die weitere Verfahrensweise ist in Beispiel 1 beschrieben. Es wurde chromosomale DNA mit den in Abbildung 3 dargestellten Restriktionsschnittstellen erhalten. Das chromosomale DNA Fragment wurde isoliert, mit dem in der DE-Patentanmeldung P 37 37 729.9 beschriebenen shuttle-Vektor pZ1 ligiert, und damit C. glutamicum ATCC 14310 transformiert. Die Threonindehydrataseaktivität dieses rekombinanten Stammes betrug 0,8 U/mg Protein, wogegen der Ausgangsstamm eine Aktivität von 0,01 U/mg Protein enthält. Der das Plasmid mit der Wildtyp DNA-Sequenz enthaltende Stamm Corynebacterium glutamicum ATCC 14310 wurde unter der Nummer DSM 5665 hinterlegt.

### Beispiel 4

### Steigerung der L-Isoleucinbildung durch erhöhte Threonindehydrataseaktivität.

In einem 500 ml Erlenmeyerkolben mit 2 Schikanen wurden 60 ml einer Nährlösung mit folgender Zusammensetzung gegeben:
40 g/l Glukose x H₂O
20 g/l Ammoniumsulfat
0,5 g/l Kaliumdihydrogensulfat
0,5 g/l Dikaliumhydrogensulfat
0,25 g/l Magnesiumsulfat
10 mg/l FeSO₄ x 7 H₂O
10 mg/l MnSO₄ x 4 H₂O
1 mg/l ZnSO₄ x 7 H₂O
0,2 mg/1 CuSO₄
20 g/l CaCO₃
Glukose wurde getrennt sterilisiert, CaCO₃ trocken sterilisiert (8 Stunden bei 150 °C) und beides der Nährlösung steril zugesetzt.

Die Fermentation wurde mit einer 16 Stunden alten Vorkultur von C. glutamicum DSM5665 angeimpft, bezw. mit C. glutamicum ATCC 14310, sodaß die Anfangszelldichte einer Optischen Dichte bei 600 nm von 0,5 bis 0,8 entsprach. Die Hauptkultur wurde bei 30°C und 140 rpm inkubiert.

Nach 72 Stunden wurden folgende Werte bestimmt:

| Stammm | mmol/l L-Isoleucin |
|---|---|
| ATCC 14310 | 4 |
| DSM 5665 | 13 |

### Beispiel 5

### Steigerung der L-Isoleucinbildung durch erhöhte Acetohydroxysäuresynthase- und Isomeroreduktaseaktivität.

Es wurde wie in Beispiel 4 verfahren, jedoch enthielt das Medium noch zusätzlich 150 mmol/1 D,L-2-Hydroxybutyrat und die Fermentation wurde mit dem Stamm DSM 5664 ausgeführt.

Nach 72 Stunden wurden folgende Werte bestimmt:

| Stamm | L-Isoleucin (mmol/l) |
|---|---|
| ATCC 13032 | 16 |
| DSM 5664 | 36 |

Brauchbare Plasmide oder Phagen wie insbesondere pZ1, pJC1, pSA77 oder BL1 sind in Mol.Gen. Genet. 220 (1989) 478-400, Appl. Environ. Microbiol. 55 (1989) 684-688, Bio/Technology 5 (1987) 137-146, J. Gen. Virol, 71 (1990) 1629-1633, J. Gen. Microbiol. 136 (1990) 567-571 bzw. Appl. Environ. Microbiol. 54 (1988) 1466-1474 beschrieben.

In den beigefügten Zeichnungen zeigen :
Figur 1 Restriktionskarte von pCC1 (Isomeroreduktasegen, ilvC, aus Corynebacterium glutamicum im Vektor pBR322).

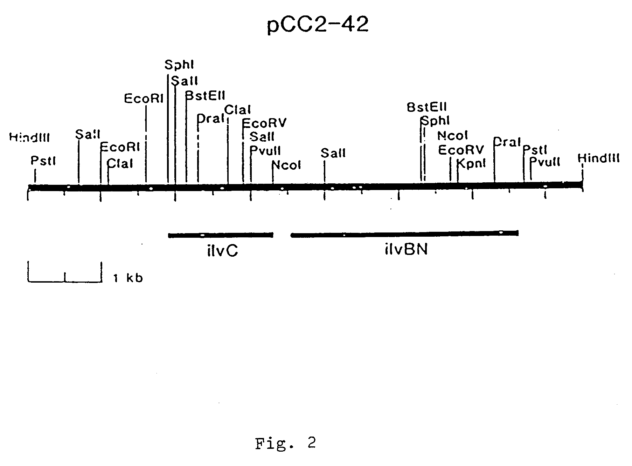

Figur 2 Restriktionskarte des 7,5 kb-HindIII-Fragments chromosomaler DNA aus Corynebacterium glutamicum, das für die Acetohydroxysäuresynthase, ilvBN, kodiert.
Figur 3 Restriktionskarte von pCC4-8, dem Plasmid, das das klonierte Threonindehydratasegen aus Corynebacterium glutamicum enthält.

## Patentansprüche

1. Verfahren zur Herstellung von L-Isoleucin durch Züchtung eines transformanten Mikroorganismus der Gattung Corynebacterium oder Brevibacterium in einem geeigneten Nährmedium und Isolierung von L-Isoleucin aus dem Medium,
**dadurch gekennzeichnet,**
daß man Mikroorganismen verwendet, die eine rekombinante DNA aus Vektor-DNA und für Threonindehydratase und Acetohydroxysäuresynthase kodierenden DNA-Sequenzen aufweist.

2. Verfahren nach Anspruch 1,
**dadurch gekennzeichnet,**
daß die rekombinante DNA zusätzlich eine für Isomeroreduktase kodierende DNA-Sequenz aufweist.

3. In Corynebacterium oder Brevibacterium replizierbare rekombinante DNA aus Vektor-DNA und für Threonindehydratase, Acetohydroxysäuresynthase und ggf. Isomeroreduktase kodierenden DNA-Sequenzen.

4. Rekominante DNA nach Anspruch 3,
**dadurch gekennzeichnet,**
daß die DNA-Sequenzen aus Corynebacterium glutamicum ATCC 13 032 (entsprechend DSM 20 300) oder Brevibacterium flavum ATCC 13 826 stammen.

5. Rekombinante DNA nach Anspruch 3,
**dadurch gekennzeichnet,**
daß die DNA-Sequenzen aus Mutanten von Corynebacterium oder Brevibacterium mit gesteigerter Gen-Expression oder Unempfindlichkeit gegenüber einer Feed back Hemmung, insbesondere für die Threonindehydratase und/oder Acetohydroxysäuresynthase isoliert sind, insbesondere aus Corynebacterium glutamicum mit der Hinderlegungs-Nr. DSM 5659.

6. Rekombinante DNA nach einer der Ansprüche 3 bis 5,
**gekennzeichnet durch**
aus Bakterien der Gattung Corynebacterium oder Brevibacterium stammende Plasmide oder Phagen, insbesondere pZ1, pJC1, pSA77 oder BL1.

7. Mit rekombinanter DNA nach einem der Ansprüche 3 bis 6 transformierte L-Isoleucin produzierende Mikroorganismen der Gattung Corynebacterium oder Brevibacterium.

## Claims

1. A method of preparing L-isoleucine by cultivating a transformant microorganism of the genus Corynebacterium or Brevibacterium in a suitable nutrient medium and isolating L-isoleucine from the medium, characterised in that microorganisms are used which have a recombinant DNA comprising vector DNA and DNA sequences which are coding for threonine dehydratase and acetohydroxyacid synthase.

2. A method according to claim 1, characterised in that the recombinant DNA additionally comprises a DNA sequence which is coding for isomeroreductase.

3. A DNA which is replicably recombinant in Corynebacterium or Brevibacterium and which comprises vector DNA and DNA sequences which are coding for threonine dehydratase, acetohydroxyacid synthase and optionally isomeroreductase.

4. A recombinant DNA according to claim 3, characterised in that the DNA sequences originate from Corynebacterium glutamicum ATCC 13 032 (corresponding to DSM 20 300) or Brevibacterium flavum ATCC 13 826.

5. A recombinant DNA according to claim 3, characterised in that the DNA sequences are isolated from mutants of Corynebacterium or Brevibacterium, particularly from Corynebacterium glutamicum with the restriction number DSM 5659, with enhanced gene expression or insensitivity to feedback inhibition, particularly for threonine dehydratase and/or acetohydroxyacid synthase.

6. A recombinant DNA according to any one of claims 3 to 5, characterised by plasmids or phages originating from bacteria of the genus Corynebacterium or Brevibacterium, particularly pZ1, pJC1, pSA77 or BL1.

7. Microorganisms of the genus Corynebacterium or Brevibacterium transformed with recombinant DNA according to any one of claims 3 to 6 and producing L-isoleucine.

## Revendications

1. Procédé de préparation de L-isoleucine par culture d'un micro-organisme transformant de type Corynebacterium ou Brevibacterium dans un milieu nutritif approprié et isolement de la L-isoleucine du milieu, caractérise en ce qu'on utilise des micro-organismes qui présentent un ADN recombinant formé d'un vecteur ADN et de séquences d'ADN codant pour la déshydratase de thréonine et la synthase d'acide hydroxyacétique.

2. Procédé selon la revendication 1, caractérisé en ce que l'ADN recombinant présente en outre une séquence d'ADN codant pour l'isoméroréductase.

3. ADN recombinant réplicable dans la souche Corynebacterium ou Brevibacterium et constitué d'un vecteur ADN et de séquences d'ADN codant pour la déshydratase de thréonine, la synthase d'acide hydroxyacétique et éventuellement l'isoméroréductase.

4. ADN recombinant selon la revendication 3 caractérisé en ce que les séquences d'ADN proviennent de la souche Corynebacterium glutamicum ATCC 13 032 (correspondant au DSM 20 300) ou de la souche Brevibacterium flavum ATCC 13 826.

5. ADN recombinant selon la revendication 3, caractérisé en ce que les séquences d'ADN sont isolées de mutants de Corynebacterium ou de Brevibacterium avec une expression du gène accrue ou une plus grande insensibilité vis-à-vis d'une inhibition de rétro-action, en particulier pour la déshydratase et/ou la synthase d'acide hydroxyacétique, en particulier à partir de Corynebacterium glutamicum de n^{o} de dépôt DSM 5659.

6. ADN recombinant selon l'une quelconque des revendications 3 à 5, caractérisé par des plasmides ou phages issus de bactéries de type Corynebacterium ou Brevibacterium, en particulier pZ1, pJC1, pSA77 ou BL1.

7. Micro-organismes de type Corynebacterium ou Brevibacterium produisant de la L-isoleucine transformée par de l'ADN recombinant selon l'une quelconque des revendications 3 à 6.
